# EUROPÄISCHE PATENTSCHRIFT

(11) **EP 4 245 256 B1**
(45) Veröffentlichungstag und Bekanntmachung des Hinweises auf die Patenterteilung: **22.04.2026**
(21) Anmeldenummer: 23162276.2
(22) Anmeldetag: 16.03.2023
(51) Int. Cl.: A61F 2/00, A61F 2/24

(54) **ANPASSUNGSFÄHIGE HÜLLE FÜR EIN MEDIZINISCHES IMPLANTAT**
CONFORMABLE SHEATH FOR A MEDICAL IMPLANT
GAINE CONFORMABLE POUR IMPLANT MEDICAL

(30) Priorität: 16.03.2022 DE 102022202613
(43) Veröffentlichungstag der Anmeldung: 20.09.2023
(73) Patentinhaber: AdjuCor GmbH, 81673 München (DE)
(72) Erfinder: Wohlfarth, Robert, 66123 Saarbrücken (DE); Kratzer, Claus Johann, 84416 Taufkirchen (DE); Maier, Andreas, 85567 Grafing bei München (DE); Wildhirt, Stephen Manuel, 82335 Berg (DE)
(74) Vertreter: Peterreins Schley

(56) Entgegenhaltungen:
- WO-A1-01/67985
- WO-A1-2005/072649
- WO-A1-2016/071823
- WO-A1-2019/081766
- WO-A1-2022/174856
- US-A1- 2007 122 590
- US-A1- 2021 052 367

## Beschreibung

### Technisches Gebiet

Die vorliegende Erfindung betrifft eine flexible Hülle für ein medizinisches Implantat, die eine hohe geometrische Anpassungsfähigkeit aufweist. Insbesondere können sich die erfindungsgemäßen flexiblen Hüllen sich ändernden Formen und Geometrien des Implantationsortes anpassen.

### Hintergrund der Erfindung

Medizinische Implantate werden in der Regel auf die individuellen Erfordernisse eines Patienten hinsichtlich Größe und Geometrie abgestimmt. Insbesondere für medizinische Implantate, die mit Organen wechselwirken, ist eine fortdauernde Passgenauigkeit für eine sichere Funktion wichtig. Dies trifft auch auf Implantate zu, die eine Hülle haben, wie zum Beispiel Stent-Grafts oder organumhüllende Implantate (z.B. eine Herzunterstützungsvorrichtung). Ein Beispiel eines Herzunterstützungsvorrichtung ist aus WO 01/67985 bekannt.

Die meisten medizinischen Implantate können sich einer nach der Implantation auftretenden Größen- oder Geometrieänderung des Implantationsortes nicht anpassen. Dies kann dazu führen, dass die Implantate nicht mehr passen, wodurch deren Funktion beeinträchtigt wird. Ursächlich für eine Formveränderung des Implantationsortes können unter anderem ein fortschreitender Krankheitsverlauf, ein eintretender Therapieerfolg, tageszeitliche Schwankungen oder Wachstum im Allgemeinen sein.

Die Aufgabe der vorliegenden Erfindung ist es ein geometrisch anpassungsfähiges Implantat mit einer Hülle bereitzustellen.

### Zusammenfassung der Erfindung

Die vorliegende Erfindung betrifft verschiedene Ausführungsformen für eine anpassungsfähige flexible Hülle für ein Herzunterstützungsvorrichtung.

Eine erste erfindungsgemäße Ausführungsform betrifft eine flexible Hülle, die eine auxetische Musterung in Form von Schnitten und/oder Wellungen, umfasst, die die Anpassungsfähigkeit der Hülle vergrößern. Die auxetische Musterung der Hülle kann ein auxetisches Materialverhalten induzieren. Die Hülle kann sich bei einer uniaxialen Zugbeanspruchung auch quer zur Zugrichtung ausdehnen. Auxetische Materialien sind zum Beispiel aus US 2021/052367, WO 2016/071823, WO 2005/072649 and WO 2019/081766 bekannt.

Die auxetische Musterung kann rotierende Dreiecke umfassen. Die auxetische Musterung kann eine Vielzahl von Y-förmigen Schnitten oder Y-förmigen Wellungen umfassen. Die Y-förmigen Schnitte können eindimensionale Einschnitte umfassen. Die Y-förmigen Schnitte können flächige Ausschnitte umfassen. Die flächigen Ausschnitte können abgerundete Ecken umfassen. Die Schnitte oder Wellungen können Sollbruchstellen definieren. Jeder der drei Schenkel eines Y-förmigen Schnittes oder einer Y-förmigen Wellung kann die gleiche Länge haben. Die Schenkel eines Y-förmigen Schnittes oder einer Y-förmigen Wellung können eine unterschiedliche Länge haben. Die Mehrzahl der Y-förmigen Schnitte oder Y-förmigen Wellungen kann vorwiegend die gleiche Schenkellänge aufweisen. Die Mehrzahl der Y-förmigen Schnitte oder Y-förmigen Wellungen kann vorwiegend eine unterschiedliche Schenkellänge aufweisen. Die Schenkel der Y-förmigen Schnitte oder Y-förmigen Wellungen können zwischen 1 mm und 1 cm lang sein. Der Mittelpunktabstand zwischen zwei benachbarten Y-förmigen Schnitten oder Y-förmigen Wellungen kann zwischen 0,2 cm und 2 cm betragen.

Die auxetische Musterung kann rotierende Vierecke umfassen, insbesondere kann die auxetische Musterung rotierende Rechtecke oder rotierende Quadrate umfassen. Die auxetische Musterung kann eine Vielzahl von Schnitten oder Wellungen umfassen, die derart angeordnet sind, dass sie zumindest abschnittsweise Kanten eines Viereckes definieren. Die Schnitte oder Wellungen ungefähr in einem rechten Winkel zueinander angeordnet sind. Die Schnitte können eindimensionale Einschnitte umfassen. Die Schnitte können flächige Ausschnitte umfassen. Die flächigen Ausschnitte können abgerundete Ecken umfassen. Die Schnitte oder Wellungen können Sollbruchstellen definieren. Die Schnitte oder Wellungen können die gleiche Länge haben. Die Schnitte oder Wellungen können eine unterschiedliche Länge haben. Die Schnitte oder Wellungen können zwischen 1 mm und 1 cm lang sein. Die Hülle kann eine longitudinale Achse haben und zumindest ein Teil der Schnitte können in der gleichen Richtung wie die longitudinale Achse angeordnet sein. Die Hülle kann eine longitudinale Achse haben und zumindest ein Teil der Schnitte kann in einem Winkel von 5° bis 45° zur longitudinalen Achse angeordnet sein. Die Hülle kann eine homogene auxetische Musterung aufweisen. Die Hülle kann eine heterogene auxetische Musterung aufweisen. Die auxetische Musterung kann rotierende Dreiecke und rotierende Vierecke umfassen. Die Hülle kann Teil einer Herzunterstützungsvorrichtung sein. Die Hülle kann mechanisch expandierbare Einheiten umfassen. Die Hülle kann ausgelegt sein ein Organ des Körpers zumindest teilweise zu umschließen. Die Hülle kann ausgelegt sein ein Herz zumindest teilweise zu umschließen. Die Hülle kann dazu ausgelegt sein, ein Organ zu umschließen, wobei die auxetische Musterung der Hülle ausgelegt ist, eine Größenänderung des Organs zu kompensieren.

Eine zweite Ausführungsform betrifft eine flexible Hülle für ein medizinisches Implantat, die einen Schaum umfasst, der die Anpassungsfähigkeit der Hülle vergrößert.

Eine dritte Ausführungsform betrifft eine flexible Hülle für ein medizinisches Implantat, die eine oder mehrere Falten umfasst, die sich öffnen können, um die Anpassungsfähigkeit der Hülle zu vergrößern.

Eine vierte Ausführungsform betrifft eine flexible Hülle für ein medizinisches Implantat, wobei die Hülle eine Netzstruktur umfasst, die die Anpassungsfähigkeit der Hülle vergrößert.

Eine fünfte Ausführungsform betrifft eine flexible Hülle für ein medizinisches Implantat, wobei die Hülle mehrere Schichten unterschiedlicher Materialsteifigkeit umfasst, wobei sich die Schichten auflösen können, um die Anpassungsfähigkeit der Hülle zu vergrößern.

Eine sechste Ausführungsform betrifft eine flexible Hülle für ein medizinisches Implantat, wobei die Hülle ein viskoelastisches Material umfasst, das die Anpassungsfähigkeit der Hülle vergrößert.

Eine siebte Ausführungsform betrifft eine flexible Hülle für ein medizinisches Implantat, wobei die Hülle eine absorbierbare Füllung umfasst, die die Anpassungsfähigkeit der Hülle vergrößert.

Die Ausführungsformen werden im Folgenden näher beschrieben.

### Übersicht der Figuren

**Figur 1a****-d** zeigt verschiedene auxetische Muster.
**Figur 2a****-b** zeigt ein auxetisches Muster mit rotierenden Dreiecken.
**Figur 3** zeigt das Verhalten eines auxetisches Muster mit rotierenden Dreiecken während einer uniaxialen Zugbeanspruchung.
**Figur 4a****-b** zeigt ein auxetisches Muster mit rotierenden Vierecken.
**Figur 5** zeigt ein Detail eines auxetischen Musters in Form von rotierenden Dreiecken.
**Figur 6** zeigt verschiedene Anordnungen eines auxetischen Musters in Form von rotierenden Dreiecken.
**Figur 7** zeigt eine Hülle mit einem auxetischen Muster in Form von flächigen Schnitten.
**Figur 8** zeigt eine Hülle mit einem auxetischen Muster in Form von Wellungen.
**Figur 9** zeigt eine Hülle, die vorwiegend aus einem Schaum besteht.
**Figur 10** zeigt eine Hülle, die einen Schaum und eine Folie umfasst.
**Figur 11a****-c** zeigt eine Hülle mit Falten.
**Figur 12** zeigt das Aufreißen von Befestigungen mit Hilfe eines Ballonkatheters.
**Figur 13** zeigt eine Hülle in Form einer Netzstruktur mit Sicherungen.
**Figur 14** zeigt eine Hülle mit mehreren Schichten unterschiedlicher Materialsteifigkeit.
**Figur 15** zeigt eine Hülle mit einem viskoelastischem Material.

### Detaillierte Beschreibung

Ausführungsformen werden im Folgenden unter Bezugnahme auf die Figuren näher beschrieben. Die Erfindung wird durch die Ansprüche definiert. Die flexiblen Hüllen sind für jegliches medizinisches Implantat mit einer Hülle anwendbar. Die Hüllen sind insbesondere für Implantate geeignet, die Organe (z.B. Lunge, Herz, Leber, Magen, Dünndarm, Dickdarm, Milz, Niere, Gallenblase, Bauchspeicheldrüse) zumindest teilweise umhüllen. Die Hüllen sind auch geeignet, Implantate wie beispielsweise Stent-Grafts oder Brustimplantate zu umhüllen. Im Folgenden werden die verschiedenen erfindungsgemäßen Ausführungsformen in Bezug auf eine Herzunterstützungsvorrichtung mit einer Hülle beschrieben, die dazu ausgelegt ist, ein Herz zumindest teilweise zu umschließen.

Eine geometrisch anpassungsfähige Implantatshülle ist insbesondere dann von Nutzen, wenn sich das zu umhüllende Organ (beispielsweise das Herz oder die Leber) im Verlauf der Zeit in seiner Größe oder Geometrie verändert. Formveränderungen können infolge eines Krankheitsverlaufes auftreten (z.B. in Folge fortschreitender Herzinsuffizienz). Formveränderungen können auch infolge eines Therapieerfolges auftreten (z.B. kann ein Herz sich nach Ausheilung einer akuten Myokarditis verkleinern). Formveränderungen können auch zyklisch auftreten (z.B. während des dynamisch stattfindenden Herzzyklus). Formveränderungen können auch durch tageszeitliche Schwankungen auftreten (z.B. Herzgeometrieveränderung durch Änderung des Volumenstatus durch Trinken oder Urinieren). Formveränderungen können auch durch Wachstum jeglicher Art auftreten (z.B. kindliches Herzwachstum oder Wucherungen von Organen wie beispielsweise der Leber oder Bauchspeicheldrüse). Schließlich sind geometrisch anpassungsfähige Implantatshüllen auch dann von Vorteil, wenn nur eine begrenzte Anzahl von Implantatsformen zur Verfügung gestellt werden kann (z.B. Verbesserung der individuellen Passgenauigkeit von Hüllen für standardisierte Herzformen).

Bei der Anwendung im pädiatrischen Bereich verhindern die erfindungsgemäßen anpassungsfähigen Implantatshüllen eine Wachstumsrestriktion des umhüllten Organs, z.B. des Herzens. Das heißt, dass durch die Anpassungsfähigkeit der Hülle das kindliche Wachstum des Herzens nicht eingeschränkt wird. Über eine Implantationsdauer von 12 bis 18 Monaten kann so der zu erwartende Volumenzuwachs des Herzens von etwa 20-25% ermöglicht werden. Die erfindungsgemäßen Hüllen können ein Organ (z.B. das Herz) oder Implantat entweder ganz oder auch nur teilweise umschließen. Es können auch nur Abschnitte einer Hülle mit den erfindungsgemäßen anpassungsfähigen Eigenschaften versehen werden. Dies kann insbesondere dann von Vorteil sein, wenn verschiedene Eigenschaften entlang der Hülle gewünscht sind.

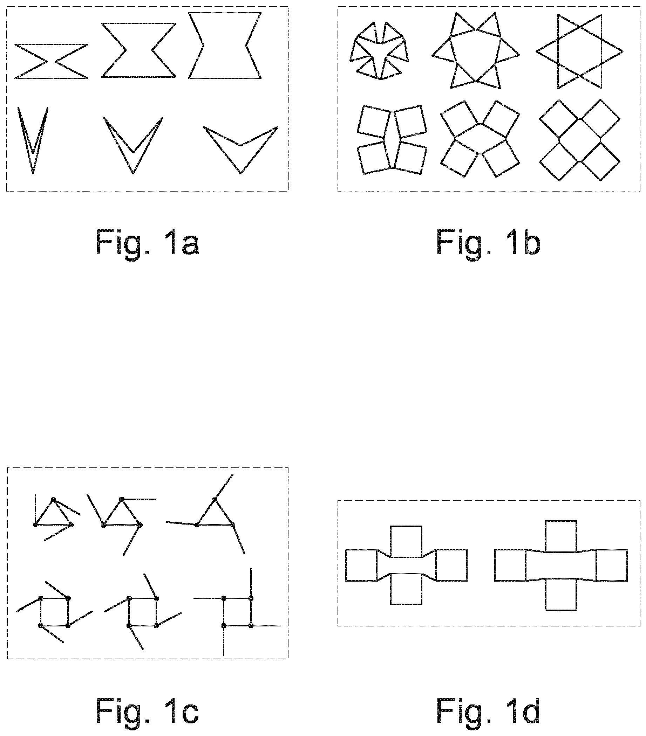

Eine erste erfindungsgemäße Ausführungsform betrifft eine flexible Hülle für ein medizinisches Implantat, wobei die Hülle eine auxetische Musterung umfasst, die die Anpassungsfähigkeit der Hülle vergrößert. Die auxetische Musterung kann beispielsweise in Form von Schnitten und/oder Wellungen vorliegen. Das auxetische Muster ermöglicht der Hülle adaptiv einer Formänderung zu folgen (beispielsweise einer Organgrößenänderung wie z.B. beim kindlichen Herzwachstum). Generell zeichnet sich auxetisches Material durch eine negative Querkontraktionszahl aus, d.h. bei uniaxialer Zugbeanspruchung einer Probe dehnt sich diese im Gegensatz zu den meisten gängigen Materialien auch quer zur Zugrichtung aus. Die im Folgenden gezeigten Musterungen sind beispielhaft und induzieren ein derartiges auxetisches Materialverhalten und werden daher auch auxetische Musterungen genannt. Ein auxetisches Muster kann unter anderem durch einspringende Strukturen (**Figur 1a**), rotierende Elemente (**Figur 1b**), chirale Strukturen (**Figur 1c**) und Elemente mit Ligamenten (**Figur 1d**) erreicht werden. Allen gemeinsam ist, dass eine Dehnung in die Längs- bzw. Querrichtung nicht mit einer Kontraktion oder Einschnürung des Materials in den Richtungen senkrecht zur Dehnungsrichtung einhergeht. Das heißt, eine Dehnung in die Längs- bzw. Querrichtung kann auch mit einer Dehnung in der Quer- bzw. Längsrichtung einhergehen. Die Materialien können sich in der Fläche, wo die auxetische Musterung vorliegt, in den unterschiedlichen Richtungen unabhängig von der anderen Richtung ausdehnen oder kontrahieren.

So würde sich eine Hülle, welche beispielsweise einen Teil eines Herzens umschließt, ohne auxetisches Materialverhalten bei Dehnung in Umfangsrichtung in der Herzlängsachse zusammenziehen. Die Hülle würde dabei in Längsrichtung kürzer werden und das Implantat passt nicht mehr zur Geometrie des Herzens. Die Hülle ohne auxetisches Materialverhalten könnte damit einer gleichmäßigen, natürlichen Größenausdehnung des Herzens in allen Richtungen nicht folgen. Die Hülle würde nicht mehr zur Geometrie des Organs passen. Bei Vorliegen eines auxetischen Materialverhaltens kann die Hülle in unterschiedlichen Richtungen unabhängige Dehnungen erlauben. Beispielsweise kann eine Hülle mit auxetischem Materialverhalten einer Vergrößerung einer Herzgeometrie sowohl in Umfangsrichtung wie auch gleichzeitig in Herzlängsrichtung folgen. Umgekehrt kann beispielsweise bei einer sich verkleinernden Herzgeometrie die Hülle sowohl in Umfangsrichtung wie auch in Längsrichtung eine Kontraktion erlauben. Die Hülle mit auxetischem Materialverhalten kann daher bei veränderlicher Herzgeometrie eine gute Passform aufrechterhalten.

Die vorliegende Erfindung ist nicht auf ein bestimmtes Muster beschränkt und die im Folgenden näher beschriebenen Musterungen sind nur zur näheren Erläuterung zu verstehen.

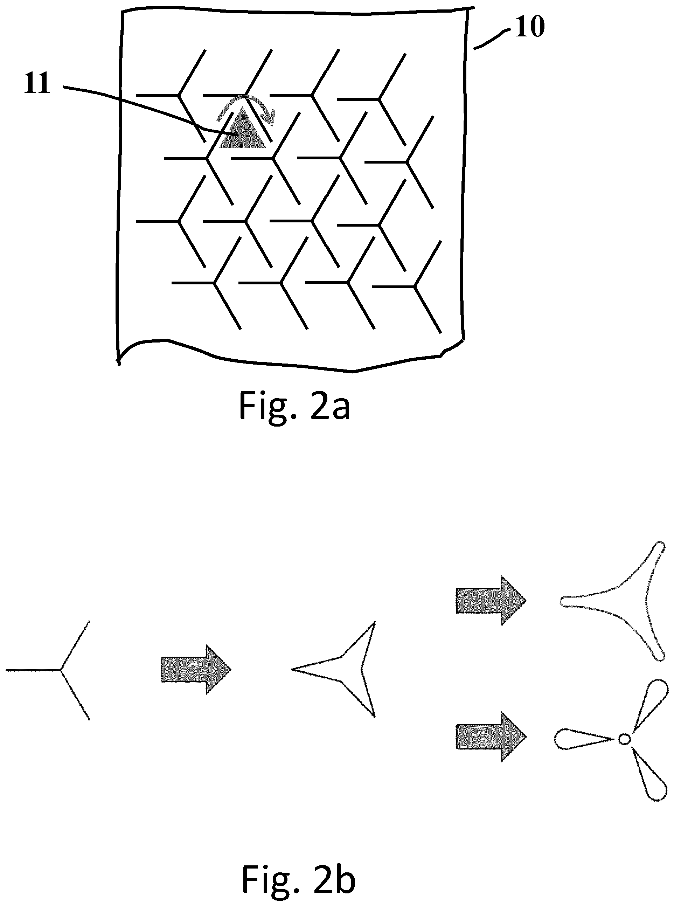

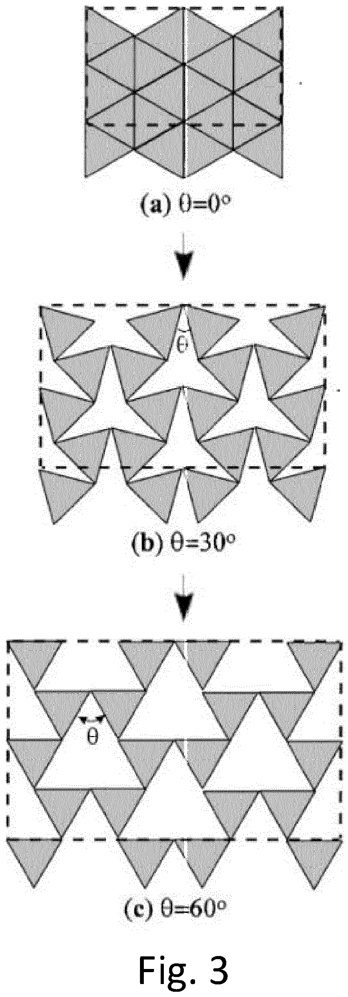

**Figur 2a** zeigt schematisch einen Ausschnitt einer Hülle (10), der eine auxetische Musterung in Form von "rotierenden Dreiecken" umfasst. Die Dreiecke können durch eine Vielzahl von Y-förmigen Schnitten oder Y-förmigen Wellungen erzeugt werden. Wie in **Figur 2b** gezeigt, können die Y-förmigen Schnitte eindimensionale Einschnitte (**Figur 2b****, links**) oder flächige Ausschnitte (**Figur 2b****, Mitte und rechts**) umfassen. Ein flächiges Ausschneiden der Musterung ist unter anderem dann von Vorteil, wenn ein Durchwachsen mit Bindegewebe angestrebt wird. Unter Ausnutzen des schnellen Remodelingprozesses des eingewachsenen Bindegewebes kann sich die Hülle dann in der Wachstumsgeschwindigkeit des Organs (z.B. dem Herz) aufdehnen oder auch eine Schrumpfung mitgehen. Die flächigen Ausschnitte können auch abgerundete Ecken umfassen **(****Figur 2b****, rechts).** Eine Abrundung der Ecken kann zu geringeren Spannungsspitzen und einer erhöhten Dauerfestigkeit der Hülle führen. Die Schnitte oder Wellungen können auch Sollbruchstellen definieren (**Figur 2c, rechts** unten). Die Sollbruchstellen können unter einer bestimmten Belastung aufreißen und zu einer Vergrößerung der Hülle führen. Will man diese Musterung als Sollbruchstelle umsetzen, nutzt man aus, dass Spannungsspitzen an Ecken auftreten und der Sollbruch vom ausgeschnittenen Kreis in der Mitte aufgefangen wird. Das Verhalten der rotierenden Dreiecke während einer uniaxialen Zugbeanspruchung ist in **Figur 3a****-c** gezeigt. Wie den **Figuren 3a****-c** zu entnehmen ist, drehen sich die dreieckigen Hüllenteilflächen in Folge der Zugbeanspruchung und vergrößern die Hülle sowohl in Zugrichtung als auch quer zur Zugrichtung.

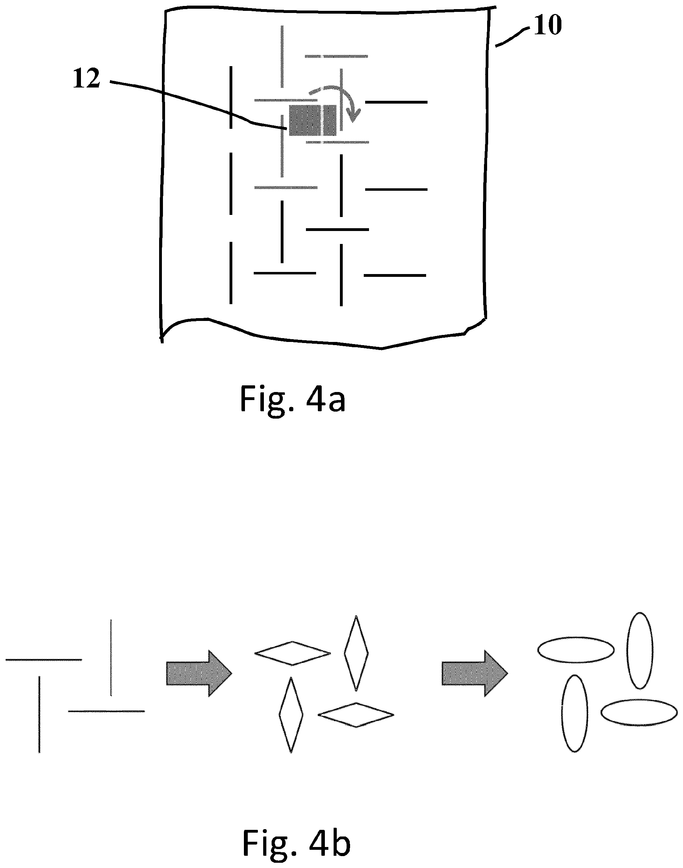

**Figur 4a** zeigt schematisch einen Ausschnitt einer Hülle (10), der eine auxetische Musterung in Form von "rotierenden Vierecken" umfasst. Die rotierenden Vierecke können unter anderem in Form von rotierenden Rechtecken oder rotierenden Quadrate vorliegen. Die auxetische Musterung kann eine Vielzahl von Schnitten oder Wellungen umfassen, die derart angeordnet sind, dass sie zumindest abschnittsweise Kanten eines Viereckes definieren. Die Schnitte oder Wellungen können im nicht-gedehnten Material ungefähr in einem rechten Winkel zueinander angeordnet sein. Wie bereits für die "rotierenden Dreiecke" beschrieben, können die Schnitte eindimensionale Einschnitte (**Figur 4b****, links**) oder flächige Ausschnitte (**Figur 4b****, Mitte und rechts**) umfassen. Die flächigen Ausschnitte können abgerundete Ecken (**Figur 4b****, rechts**) umfassen. Die Schnitte oder Wellungen können auch Sollbruchstellen definieren.

Folgender Mechanismus kann zu einer guten Adaptivität der Hülle führen, sodass diese einer Formänderung des Organs folgt. Die durch die (flächigen) Schnitte entstandenen Öffnungen erlauben eine Integration von Zellen, sodass nach der Implantation diese Schnitte auf der Hülle beispielsweise von Bindegewebe durchwachsen werden. Damit kann das Implantat mit dem Organ verwachsen. Vollzieht das Organ nun eine Formänderung, so passt sich die Hülle über ein Aufweiten oder Schließen der Öffnungen an die neue Geometrie an. Dabei führt eine Rotationsbewegung von Hüllenteilflächen (z.B. der dreieckigen Teilfläche, siehe Pfeil in **Figur 2a****,** oder der viereckigen Teilfläche, siehe Pfeil in **Figur 4a**) dazu, dass sich das Hüllenmaterial unter geringen Materialspannungen anisotrop unabhängig voneinander in beide Raumrichtungen ausdehnen kann, ein auxetisches Materialverhalten aufweist und somit zu einer Hüllenvergrößerung in beide Raumrichtungen führt (siehe hierzu auch **Figur 3**). Dabei nutzt man das häufige Remodeling von Bindegewebe aus. Bindegewebe erneuert sich nämlich in kurzen zeitlichen Abständen in einem mechanisch spannungsfreien Zustand, sodass vom Bindegewebe ein Aufweiten oder Schließen der Öffnungen mitgetragen werden kann.

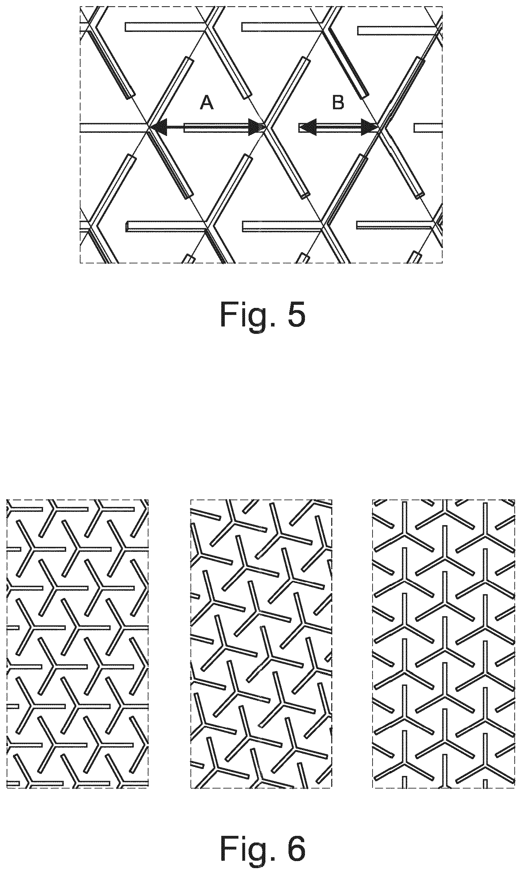

Über die Form und Gestalt der Musterung können die mechanischen Eigenschaften, insbesondere die Elastizität und das anisotrope Verhalten der Hülle nach Bedarf eingestellt werden und die Dehnung der Hülle auf Hauptwachstumsrichtungen des Organs und Hauptbelastungsrichtungen abgestimmt werden. Dabei hat man neben der Art des auxetischen Musters (einspringende Strukturen, rotierende Elemente, chirale Strukturen und Elementen mit Ligamenten, siehe **Figuren 1a****-d)** noch weitere Freiheitsgrade, die Musterung zu gestalten. So können die gewünschten Materialeigenschaften durch eine Variation der Dichte der Musterung eingestellt werden. Dazu kann wie in **Figur 5** dargestellt der Mittelpunktabstand A der Y-Schnitte und/oder Y-Wellungen variiert werden. Die Dichte kann auch bei den rotierenden Vierecken durch eine Variation des Abstands der Schnitte und/oder Wellungen eingestellt werden. Die Eigenschaften können auch durch eine Veränderung der Länge der Schnitte eingestellt werden. Durch Variation der Schenkellänge B der Y-Schnitte und/oder Y-Wellungen werden steifere oder elastischere Eigenschaften hervorgerufen. Gleiches gilt auch für die rotierenden Vierecke.

Beim Muster der rotierenden Dreiecke können die drei Schenkel eines Y-förmigen Schnittes oder einer Y-förmigen Wellung die gleiche Länge oder eine unterschiedliche Länge haben. Die Mehrzahl der Y-förmigen Schnitte oder Y-förmigen Wellungen können vorwiegend die gleiche oder eine unterschiedliche Schenkellänge aufweisen. Die Schenkel der Y-förmigen Schnitte oder Y-förmigen Wellungen können zwischen 1 mm und 1 cm lang sein. Der Mittelpunktabstand zwischen zwei benachbarten Y-förmigen Schnitten oder Y-förmigen Wellungen kann zwischen 0,2 cm und 2 cm betragen. Die Musterung kann auch zumindest teilweise in Bezug auf die longitudinale Achse ausgerichtet sein. So kann ein Schenkel der Y-Strukturen in Richtung der longitudinalen Achse der Hülle verlaufen (**Figur 6****, links**). Es ist auch möglich, die Y-Strukturen zu drehen, beispielsweise um 15° (**Figur 6****, Mitte**) oder 30° (**Figur 6****, rechts**) oder 45° oder einen beliebigen anderen Winkel, um die gewünschten Materialeigenschaften zu erzielen.

Beim Muster der rotierenden Vierecke können die Schnitte oder Wellungen die gleiche oder eine unterschiedliche Länge haben. Die Schnitte oder Wellungen können zwischen 1 mm und 1 cm lang sein. Zumindest ein Teil der Schnitte kann in der gleichen Richtung wie die longitudinale Achse angeordnet sein, wenn die Hülle im nicht-gedehnten Zustand ist. Zumindest ein Teil der Schnitte kann in einem Winkel von 5° bis 45° zur longitudinalen Achse angeordnet sein.

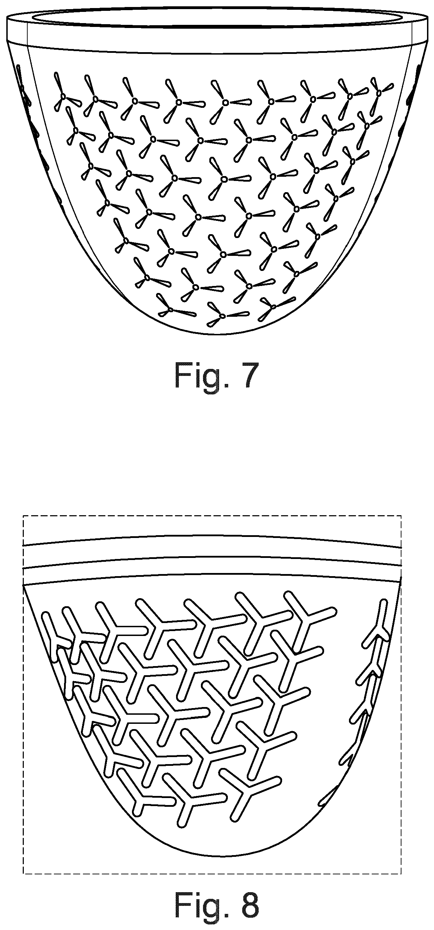

Eine beispielhafte Hülle zur zumindest teilweisen Umhüllung eines Herzens ist in **Figur 7** dargestellt. Diese Hülle umfasst ein auxetisches Muster in der Form von rotierenden Dreiecken, wobei die Y-Strukturen als abgerundete flächige Ausschnitte mit Sollbruchstellen ausgestaltet sind (vgl. **Figur 2b** **rechts unten**). Diese Hülle zeigt eine sehr gute Anpassungsfähigkeit an das menschliche Herz und kann Formänderungen des Herzens gut folgen.

Anstelle der oben beschriebenen Schnitte können sämtliche Formen auch als Wellungen ausgestaltet sein. Ist ein Durchwachsen der Hülle nicht vorgesehen, kann es vorteilhaft sein, statt der (flächigen) Schnitte das auxetische Muster in Form von Wellungen auszugestalten.

Diese Wellungen können in das Hüllenmaterial eingeprägt werden und verleihen der Hülle ein auxetisches Materialverhalten. Es ist auch möglich eine Hülle mit Schnitten und Wellungen zu versehen, um gewünschte Materialeigenschaften zu erhalten. Ein Beispiel für eine Hülle zur zumindest teilweisen Umhüllung eines Herzens mit Y-Strukturen in Form von Wellungen ist in der **Figur 8** gezeigt. Die Hülle zeigt ein sehr gutes auxetisches Verhalten und die Wellungen können ein Aufreißen der Hülle verhindern.

Erfindungsgemäß kann die die Hülle eine homogene auxetische Musterung aufweisen, d.h. die Musterung ist entlang der Hülle mehr oder weniger gleich. Es ist auch möglich eine heterogene auxetische Musterung bereitzustellen. Es können auch verschiedene auxetische Musterungen miteinander kombiniert werden, beispielsweise rotierende Dreiecke mit rotierenden Vierecken. Eine Kombination mit den eingangs beschriebenen einspringenden Strukturen (**Figur 1a**), rotierenden Elementen (**Figur 1b**), chiralen Strukturen (**Figur 1c**) und Elementen mit Ligamenten (**Figur 1d**) ist ebenfalls möglich.

Die erfindungsgemäße Hülle kann Teil einer Herzunterstützungsvorrichtung sein. Die Herzunterstützungsvorrichtung kann mechanisch expandierbare Einheiten umfassen. Die Hülle kann ausgelegt sein, ein Organ des Körpers zumindest teilweise zu umschließen. Die Hülle kann ausgelegt sein ein Herz zumindest teilweise zu umschließen. Die Hülle kann dazu ausgelegt sein, ein Organ zu umschließen, wobei die auxetische Musterung der Hülle ausgelegt ist, eine Größenänderung des Organs zu kompensieren.

Als Materialien für eine Hülle mit einer auxetischen Musterungen kommen insbesondere Polymere, wie beispielsweise Polyurethan, Polyethylenterephthalat (PET) und Silikon in Frage.

Ein Vorteil der beschriebenen auxetischen Muster ist, dass eine Materialeigenschaft (z.B. Dehnbarkeit) eingestellt werden kann, ohne dass ein Materialwechsel erforderlich ist. Die beschriebenen Hüllen müssen auch nicht verdickt oder verdünnt werden, um die Dehnbarkeit passgenau einzustellen. Die beschriebenen Musterungen erlauben es, ein anisotropes Materialverhalten einzubringen. Insbesondere die Ausführungsformen mit Schnitten ermöglichen eine gute Gewebeverwachsung. Die flächigen Ausschnitte erlauben es ferner, eine Schrumpfung mitzugehen. Ein weiterer Vorteil ist, dass die gezeigten Hüllen unkompliziert durch Stanzen und Prägen hergestellt werden können.

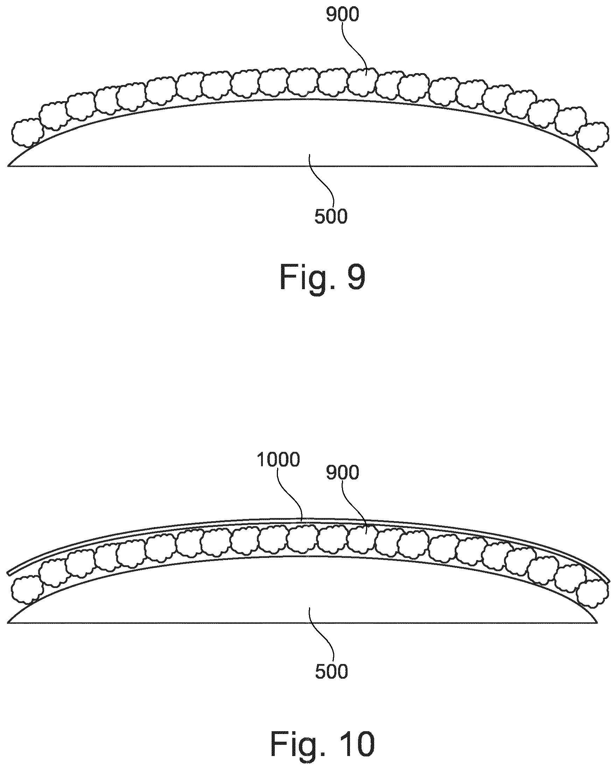

Eine zweite Ausführungsform betrifft eine flexible Hülle für ein medizinisches Implantat die einen Schaum, umfasst, der die Anpassungsfähigkeit der Hülle vergrößert. Dabei kann die Hülle wie in **Figur 9** schematisch gezeigt vorwiegend oder ausschließlich aus dem Schaum (900) bestehen. Der Schaum kann das Herz oder insbesondere das Epikard (500) umhüllen. Der Schaum kann ausgelegt sein, von Bindegewebe durchwachsen zu werden. Da Bindegewebe sich in schnellen Abständen erneuert, wird der Schaum von der Formveränderung des Bindegewebes getrieben und geht die Formänderung des Organs (z.B. dem Herz) mit. Der Schaum kann ein offenporiger Schaum sein. Aufgrund seiner intrinsischen Eigenschaft, dass sich bei Zugbeanspruchung die Zellwände in Zugrichtung ausrichten, ist offenporiger Schaum lokal sehr dehnbar und adaptiv und schränkt deshalb Bindegewebs- und Organ-/Herzwachstum nicht ein. Der Schaum kann eine Porengröße von 20 Mikrometer bis 2 Millimeter aufweisen, insbesondere von 50 Mikrometer bis 1 mm. Der Schaum kann unter anderem aus Polyurethan, PET oder Silikon bestehen. Eine Hülle, die vorwiegend oder ausschließlich aus Schaum besteht hat eine gute Adaptivität und zeigt eine gute Gewebeverwachsung. Durch seine sehr geringe Dichte beeinflusst der Schaum die natürliche Organ-/Herzbewegung kaum.

Die flexible Hülle kann zusätzlich zum Schaum (900) auch eine Folie (1000) umfassen. Wie in **Figur 10** schematisch gezeigt, kann die Hülle ausgelegt sein, ein Organ zu umschließen, wobei der Schaum auf der organzugewandten Seite und die Folie auf der organabgewandten Seite angeordnet ist. Die Folie für die Hülle kann derart dimensioniert sein, dass sie zum Zeitpunkt der Implantation im Hinblick auf das zu unterstützende Organ/Herz überdimensioniert ist. Der entstehende Raum zwischen Folie und Epikard wird dann durch den Schaum aufgefüllt. Der Schaum kann schnell von Bindegewebe durchwachsen werden. Dadurch dass sich weicher Schaum bis zu seiner Densifikation fast ohne mechanischen Widerstand komprimieren lässt, gibt er dem Organ-/Herzwachstum nach. Auch hier wird ausgenutzt, dass der Schaum das Remodeling des Bindegewebes und somit auch die Formveränderung des Organs/Herzens mitgeht. Es wird also ein Perspektivwechsel von einem mitwachsenden Implantat hin zu einem Implantat, in das das Organ/Herz hineinwächst, vollzogen. Besonders vorteilhaft hierbei ist, dass am kompletten Implantat Organ/Herzwachstum ermöglicht wird. Die verwendete Folie stellt die im Vergleich reinen Schaumansatz (**Figur 9**) die notwendige Formstabilität zur Verfügung. Die Folie kann beispielsweise aus Polyurethan, PET oder Silikon bestehen.

Eine dritte Ausführungsform betrifft eine flexible Hülle für ein medizinisches Implantat, die eine oder mehrere Falten umfasst, die sich öffnen können, um die Anpassungsfähigkeit der Hülle zu vergrößern. Das zugrundeliegende Prinzip bei der Öffnung von Falten ist, dass in der Falte Hüllenoberfläche konserviert wird, die erst im Laufe der Implantationsdauer über eine Öffnung der Falte freigegeben wird. Somit kann sich die Implantatshülle im Laufe der Zeit vergrößern. Dabei können unterschiedliche Ansätze zur Konservierung der geschlossenen Falte erwägt werden.

Eine oder mehrere der Falten können durch einen resorbierbaren Klebstoff und/oder durch resorbierbare Nähte gesichert sein, d.h. Materialien, die mit der Zeit im Körper degenerieren. Geeignet sind unter anderem Cyanoarcrylat-basierte Klebstoffe, lösemittelbasierte Klebstoffe, Kollagenklebstoffe, einweißbasierte Klebstoffe, Glutaraldehyd-Klebstoffe. Für resorbierbare Nähte eignen sich Nähte aus Polyglykolsäure, Polydioxanon, Polyglykolsäure-Caprolacton. Es ist auch möglich, die Falten durch Nähte zu sichern, die durch mechanische Belastung aufreißen (beispielsweise infolge der langen Belastung und des Materialverschleißes). Die Falten können durch Nähte und/oder Klebstoff derart gesichert sein, dass sich diese zeitversetzt öffnen. Die eine oder mehrere Falten können durch Nähte gesichert sein, die derart angeordnet sind, dass diese operativ geöffnet werden können.

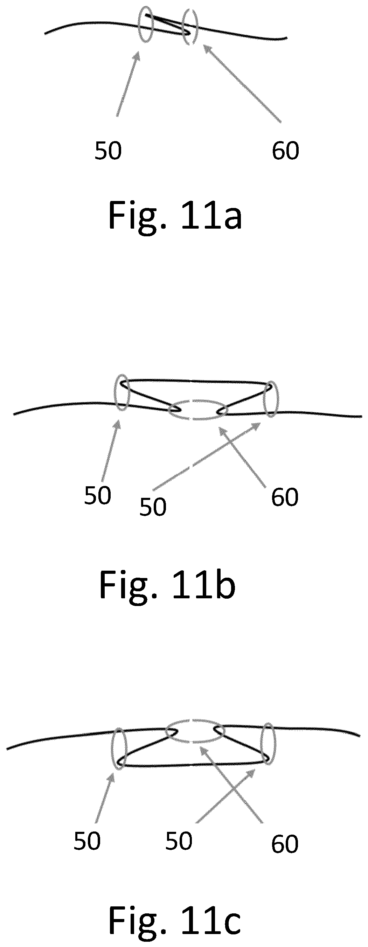

Mögliche Fixierungsoptionen der Falte durch Nähte sind in den **Figuren 11a****-c** dargestellt. Die Falte kann eine einfache Falte wie in **Figur 11a** dargestellt sein. Diese kann mit einer Typ 1 Befestigung (50) gesichert werden, die ein Abstehen der Falte verhindert. Eine Typ 2 Befestigung (60) kann die Öffnung der Falte verhindern. Die Falte kann auch wie in **Figur 11b** dargestellt eine zweifache Falte sein, die nach außen gerichtet ist. Die Falte kann auch wie in **Figur 11c** dargestellt eine zweifache Falte sein, die nach innen gerichtet ist. Es können auch verschiedene Falten und verschiedene Befestigungen kombiniert werden.

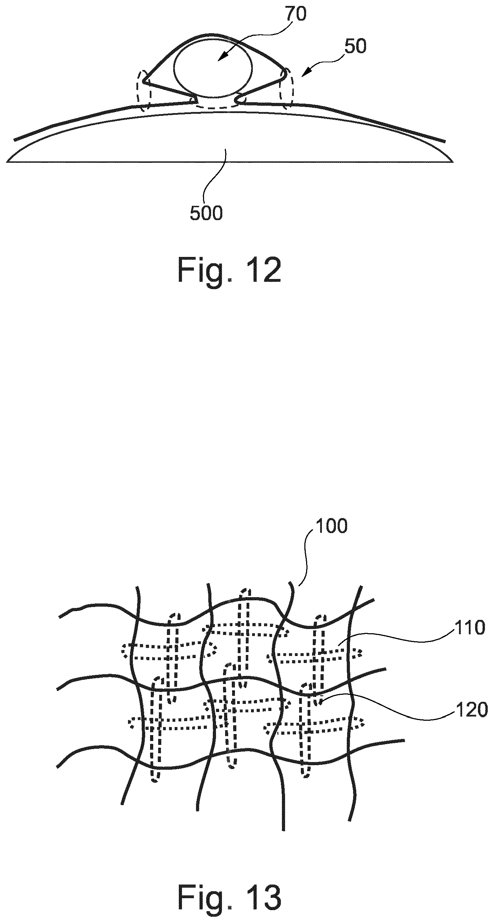

Die Falten können auch ausgelegt sein, mechanisch unter Zugbeanspruchung aufzureißen. Dies kann mit Hilfe eines in die Falte eingeführten Ballonkatheters erfolgen. Ein Aufblasen des Ballons (70) kann wie in der **Figur 12** dargestellt zum Aufreißen der Befestigung (50) führen, wobei die Befestigung in Form einer Naht oder auch in Form einer anderen Sicherung (z.B. Klebstoff) vorliegen kann.

Die Hüllen mit Falten können aus den gleichen Materialien wie die eingangs beschriebenen Hüllen mit auxetischen Mustern gefertigt sein.

Eine vierte Ausführungsform betrifft eine flexible Hülle für ein medizinisches Implantat, wobei die Hülle eine Netzstruktur umfasst, die die Anpassungsfähigkeit der Hülle vergrößert. Die Netzstruktur kann resorbierbare Strukturen wie beispielsweise resorbierbare Fäden und/oder Klemmen umfassen. Diese können derart angeordnet sein, dass sich die Netzstruktur nach deren Resorption ausdehnen kann. Beispielsweise kann ein Teil der resorbierbaren Strukturen vertikal und/oder horizontal zur longitudinalen Achse angeordnet sein. Wie in **Figur 13** zu sehen, umfasst die Netzstruktur (100) horizontale resorbierbare Strukturen (110) und vertikale resorbierbare Strukturen (120). Nach Resorption der sichernden Strukturen (110, 120) kann sich die Netzstruktur weiter ausdehnen. Die resorbierbaren Strukturen können auch in einem Winkel von 30° bis 60° zur longitudinalen Achse angeordnet sein. Mit Hilfe einer Netzstruktur mit resorbierbaren Strukturen kann ein zeitlich gesteuertes Hüllenwachstum erreicht werden. Vorteil einer Netzstruktur ist auch, dass diese eine hohe Durchlässigkeit aufweist, was zu einer guten Gewebsverwachsung führen kann. Die Netzstruktur kann aus Fäden oder Drähten hergestellt sein. Für die Netzstruktur eignen sich insbesondere Polymere wie beispielsweise Polyester, PET, PTFE, ePTFE, Polyethylen und/oder Metalle wie beispielsweise Nitinol, Stahl- und Eisenbasislegierungen, Titan und Titanlegierungen, Cobalt-Basislegierungen, Nickelbasislegierungen.

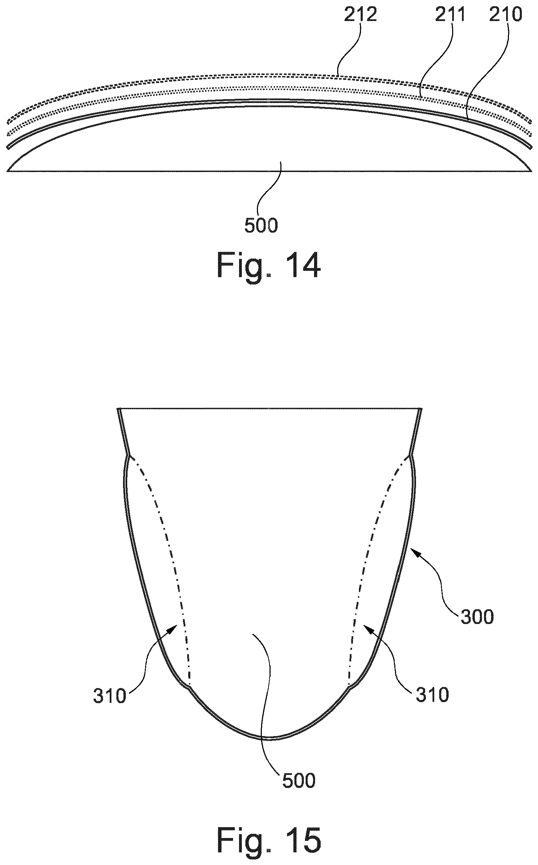

Eine fünfte Ausführungsform betrifft eine flexible Hülle für ein medizinisches Implantat, wobei die Hülle mehrere Schichten unterschiedlicher Materialsteifigkeit umfasst, wobei sich die Schichten auflösen können, um die Anpassungsfähigkeit der Hülle zu vergrößern. Die Hülle kann ausgelegt sein, dass sich die Schicht auf einer Seite zuerst auflöst. Wie in **Figur 14** gezeigt kann die Hülle eine innere stabile Schicht (210) mit niedriger Steifigkeit und eine äußere auflösbare Schicht (212) mit hoher Steifigkeit umfassen. Zwischen diesen beiden Schichten kann eine mittlere Schicht (211) mit mittlerer Steifigkeit angeordnet sein, wobei die mittlere Schicht sich langsamer als die äußere Schicht auflöst. Die Schichten können sich zeitlich versetzt von außen nach innen (z.B. infolge einer hydrolytischen Reaktion) auflösen. Die innerste Schicht ist dabei stabil, d.h. sie löst sich nicht auf, sodass sich die Hülle nie ganz auflöst. Die zunehmende Auflösung der Schichten führt dabei im Laufe der Implantationsdauer zu einer Abnahme der mittleren Steifigkeit der Hülle und somit zu einer erweiterten Dehnung der Hülle. Diese erweiterte Dehnung führt zu einer Vergrößerung der Implantatshülle, wodurch im Laufe der Zeit eine vergrößerte Organ-/Herzgeometrie unterstützt werden kann. Die Hülle kann aus zwei, drei, vier, fünf, sechs oder mehr Schichten mit verschiedenen Eigenschaften bestehen. Für die auflösbaren Schichten eignen sich Polyglykolsäure, Polydioxanon, Polyglykolsäure-Caprolacton und hydrolytisch oder oxidativ abbaubare Polyurethane wie beispielsweise Polyether- oder Polyesterurethane. Für die stabilen Schichten eignen sich nicht degenerierbare Polyurethane (z.B. Polycarbonaturethane), Polyester, insb. PET und Silikone.

Eine sechste Ausführungsform betrifft eine flexible Hülle für ein medizinisches Implantat, wobei die Hülle ein viskoelastisches Material umfasst, das die Anpassungsfähigkeit der Hülle vergrößert. Als Material eignen sich alle oben erwähnten Kunststoffe. Viskoelastisches oder auch viskoplastisches Material zeichnet sich dadurch aus, dass es bei langfristiger (oft auch nur geringer) mechanischer Belastung eine Dehnung in Form von Kriecheffekten zeigt. Aufgrund dieser kontinuierlich steigenden Dehnung wird die Implantatshülle größer, wodurch dann Organe/Herzen größer werdender Geometrie unterstützt werden können. Somit kann die Hülle beispielsweise ein kindliches Herzwachstum mitgehen. Es können nur einzelne Bereiche der Hülle vorwiegend aus dem viskoelastischen Material bestehen. Da man die größten Dehnungen aber an den Stellen der Hülle hätte, an denen die größten Materialspannungen vorliegen, aber nicht unbedingt an den Stellen, wo die größten Formadaptionen notwendig wären, ist es sinnvoll, nur einzelne Bereiche oder Streifen der Hülle aus viskoelastischem/viskoplastischem Material zu fertigen. Die Bereiche können in Form von Streifen ausgebildet sind. Das viskoelastische Material kann ein Polymer umfassen, wie beispielsweise Polyglykolsäure, Polydioxanon, Polyglykolsäure-Caprolacton, Polyether- oder Polyesterurethane und Silikone.

Eine siebte Ausführungsform betrifft eine flexible Hülle für ein medizinisches Implantat, wobei die Hülle eine absorbierbare Füllung umfasst, die die Anpassungsfähigkeit der Hülle vergrößert. Über die Verwendung von sich auflösenden Füllungen, die in die Hülle durchlässig eingeschlossen werden, kann eine zeitlich gesteuerte Dehnung der Hülle erreicht werden. Durch Auflösung der Füllung wird Raum für Organ-/Herzwachstum frei. Die Hülle kann eine Folie umfassen, die die absorbierbare Füllung zumindest teilweise umschließt. Die Folie kann Löcher umfassen, damit Körperflüssigkeiten mit der Füllung in Kontakt kommen können, um diese zu absorbieren. Die absorbierbare Füllung kann wie im Beispiel der **Figur 15** nur in Bereichen (310) vorliegen. Die Füllung kann auch in einer oder mehreren Kammern eingebracht sein. Die Hülle kann ausgelegt sein, ein Organ zumindest teilweise zu umschließen, wobei die absorbierbare Füllung auf der Innenseite der Hülle angebracht ist. Die absorbierbare Füllung kann ein Polymer umfassen, insbesondere ein Polycaprolacton (PCL), Polyglykolsäure, Polydioxanon, Polyglykolsäure-Caprolacton, degenerierbare Polyurethane (Polyesterurethan, Polyetherurethan). Die Hülle kann eine Folie aus Polyurethan umfassen, insbesondere aus thermoplastischem Polyurethan (TPU), Polyester, PET, Polyethylen, Silikon. Durch die Absorption der Füllung wird Raum für Herzwachstum frei. Die Innenseite der Füllung (oder der Kammer) kann gewellt sein. Bei einer gewellten Innenseite der Füllung kann Hüllenoberfläche gespeichert werden, die dann über die Auflösung der Füllung zeitlich gesteuert freigesetzt wird, wodurch die Implantatshülle vergrößert wird. Die Implantatshülle bleibt immer gespannt, sodass ein guter Kraftschluss über die gesamte Implantationsdauer garantiert wird.

## Patentansprüche

1. Eine flexible Hülle für ein medizinisches Implantat, wobei die Hülle Teil einer Herzunterstützungsvorrichtung ist und ausgelegt ist, das Herz eines Patienten zumindest teilweise zu umschließen, **dadurch gekennzeichnet, dass** die Hülle eine auxetische Musterung in Form von Schnitten und/oder Wellungen umfasst, die die Anpassungsfähigkeit der Hülle vergrößern.

2. Die flexible Hülle nach Anspruch 1, wobei die auxetische Musterung der Hülle ein auxetisches Materialverhalten induziert, insbesondere wobei sich die Hülle bei einer uniaxialen Zugbeanspruchung auch quer zur Zugrichtung ausdehnt.

3. Die flexible Hülle nach einem der vorhergehenden Ansprüche, wobei die auxetische Musterung rotierende Dreiecke umfasst.

4. Die flexible Hülle nach einem der vorhergehenden Ansprüche, wobei die auxetische Musterung eine Vielzahl von Y-förmigen Schnitten oder Y-förmigen Wellungen umfasst.

5. Die flexible Hülle nach Anspruch 4, wobei die Schenkel der Y-förmigen Schnitte oder Y-förmigen Wellungen zwischen 1 mm und 1 cm lang sind und/oder wobei der Mittelpunktabstand zwischen zwei benachbarten Y-förmigen Schnitten oder Y-förmigen Wellungen zwischen 0,2 cm und 2 cm beträgt.

6. Die flexible Hülle nach einem der vorhergehenden Ansprüche, wobei die auxetische Musterung rotierende Vierecke umfasst, insbesondere wobei die Musterung rotierende Rechtecke und/oder rotierende Quadrate umfasst.

7. Die flexible Hülle nach einem der vorhergehenden Ansprüche, wobei die auxetische Musterung eine Vielzahl von Schnitten oder Wellungen umfasst, die derart angeordnet sind, dass sie zumindest abschnittsweise Kanten eines Viereckes definieren.

8. Die flexible Hülle nach Anspruch 6 oder 7, wobei die Schnitte oder Wellungen ungefähr in einem rechten Winkel zueinander angeordnet sind.

9. Die flexible Hülle nach einem der vorhergehenden Ansprüche, wobei die Schnitte eindimensionale Einschnitte umfassen.

10. Die flexible Hülle nach einem der vorhergehenden Ansprüche, wobei die Schnitte flächige Ausschnitte umfassen.

11. Die flexible Hülle nach Anspruch 10, wobei die flächigen Ausschnitte abgerundete Ecken umfassen.

12. Die flexible Hülle nach einem der vorhergehenden Ansprüche, wobei die Schnitte oder Wellungen Sollbruchstellen definieren.

13. Die flexible Hülle nach einem der vorhergehenden Ansprüche, wobei die Hülle dazu ausgelegt ist, ein Organ zumindest teilweise zu umschließen und wobei die auxetische Musterung der Hülle ausgelegt ist, eine Größenänderung des Organs zu kompensieren.

## Claims

1. Flexible sheath for a medical implant, wherein the sheath is part of a cardiac-support device and is designed to at least partially enclose a patient's heart, **characterized in that** the sheath comprises an auxetic pattern in the form of cuts and/or corrugations, which increase the adaptability of the sheath.

2. Flexible sheath according to Claim 1, wherein the auxetic pattern of the sheath induces an auxetic material behaviour, in particular wherein the sheath, when exposed to uniaxial tensile stress, also expands transversely to the pulling direction.

3. Flexible sheath according to either of the preceding claims, wherein the auxetic pattern comprises rotating triangles.

4. Flexible sheath according to any one of the preceding claims, wherein the auxetic pattern comprises a multiplicity of Y-shaped cuts or Y-shaped corrugations.

5. Flexible sheath according to Claim 4, wherein the legs of the Y-shaped cuts or Y-shaped corrugations are between 1 mm and 1 cm long and/or wherein the centre-to-centre distance between two adjacent Y-shaped cuts or Y-shaped corrugations is between 0.2 cm and 2 cm.

6. Flexible sheath according to any one of the preceding claims, wherein the auxetic pattern comprises rotating quadrilaterals, in particular wherein the pattern comprises rotating rectangles and/or rotating squares.

7. Flexible sheath according to any one of the preceding claims, wherein the auxetic pattern comprises a multiplicity of cuts or corrugations, which are arranged in such a way that they define, at least in part, edges of a quadrilateral.

8. Flexible sheath according to Claim 6 or 7, wherein the cuts or corrugations are arranged approximately at right angles to one another.

9. Flexible sheath according to any one of the preceding claims, wherein the cuts comprise one-dimensional incisions.

10. Flexible sheath according to any one of the preceding claims, wherein the cuts comprise surface-area cutouts.

11. Flexible sheath according to Claim 10, wherein the surface-area cutouts comprise rounded corners.

12. Flexible sheath according to any one of the preceding claims, wherein the cuts or corrugations define predetermined breaking points.

13. Flexible sheath according to any one of the preceding claims, wherein the sheath is designed to at least partially enclose an organ and wherein the auxetic pattern of the sheath is designed to compensate for a change in size of the organ.

## Revendications

1. Gaine souple pour un implant médical, la gaine faisant partie d'un dispositif d'assistance cardiaque et étant conçue pour envelopper au moins partiellement le cœur d'un patient, **caractérisée en ce que** la gaine comprend un motif auxétique sous forme d'incisions et/ou d'ondulations, qui augmentent la conformabilité de la gaine.

2. Gaine souple selon la revendication 1, le motif auxétique de la gaine induisant un comportement auxétique du matériau, en particulier, la gaine s'étendant également transversalement par rapport à la direction de traction lors d'une sollicitation par traction uniaxiale.

3. Gaine souple selon l'une des revendications précédentes, le motif auxétique comprenant des triangles qui tournent.

4. Gaine souple selon l'une des revendications précédentes, le motif auxétique comprenant une multitude d'incisions en forme de Y ou d'ondulations en forme de Y.

5. Gaine souple selon la revendication 4, les branches des incisions en forme de Y ou des ondulations en forme de Y présentant une longueur entre 1 mm et 1 cm et/ou l'écartement entre les centres de deux incisions adjacentes en forme de Y ou ondulations adjacentes en forme de Y représentant entre 0,2 cm et 2 cm.

6. Gaine souple selon l'une des revendications précédentes, le motif auxétique comprenant des quadrilatères qui tournent, le motif comprenant en particulier des rectangles qui tournent et/ou des carrés qui tournent.

7. Gaine souple selon l'une des revendications précédentes, le motif auxétique comprenant une multitude d'incisions ou d'ondulations qui sont agencées de telle sorte qu'elles définissent au moins en partie des bords d'un quadrilatère.

8. Gaine souple selon la revendication 6 ou 7, les incisions ou les ondulations étant agencées approximativement à angle droit les unes par rapport aux autres.

9. Gaine souple selon l'une des revendications précédentes, les incisions comprenant des entailles unidimensionnelles.

10. Gaine souple selon l'une des revendications précédentes, les incisions comprenant des découpes planes.

11. Gaine souple selon la revendication 10, les découpes planes comprenant des coins arrondis.

12. Gaine souple selon l'une des revendications précédentes, les incisions ou les ondulations définissant des points destinés à la rupture.

13. Gaine souple selon l'une des revendications précédentes, la gaine étant conçue pour envelopper au moins partiellement un organe et le motif auxétique de la gaine étant conçu pour compenser une modification de la dimension de l'organe.
